(19) Europäisches Patentamt
European Patent Office
Office européen des brevets

(11) Veröffentlichungsnummer: **0 248 972**
**A2**

(12)

# EUROPÄISCHE PATENTANMELDUNG

(21) Anmeldenummer: **87100505.4**

(22) Anmeldetag: **16.01.87**

(51) Int. Cl.³: **C 07 C 87/14**
C 07 C 91/04, A 61 K 7/22

(30) Priorität: **21.02.86 DE 3605656**

(43) *Veröffentlichungstag der Anmeldung:*
**16.12.87 Patentblatt 87/51**

(84) Benannte Vertragsstaaten:
**AT BE CH DE ES FR GB IT LI LU NL SE**

(71) Anmelder: **Benckiser-Knapsack GmbH**
**Dr. Albert-Reimann-Strasse 2**
**D-6802 Ladenburg(DE)**

(72) Erfinder: **Kleemann, Stephan G., Dr. Dipl.-Chem.**
**Mozartstrasse 25**
**D-6905 Schriesheim(DE)**

(72) Erfinder: **Auel, Theodor, Dr. Dipl.-Chem.**
**Winzerstrasse 3**
**D-6803 Edingen-Neckarhausen(DE)**

(72) Erfinder: **Dany, Franz-Josef, Dr. Dipl.-Chem.**
**Heddinghovener Strasse 47**
**D-5042 Erftstadt(DE)**

(74) Vertreter: **Grussdorf, Jürgen, Dr. et al,**
**Patentanwälte Zellentin & Partner Rubensstrasse 30**
**D-6700 Ludwigshafen(DE)**

## (54) Ammoniummonofluorphosphate, Verfahren zu ihrer Herstellung und ihre Verwendung.

(57) Die vorliegende Erfindung betrifft Ammoniummonofluorophosphate der allgemeinen Formel I

$$R_1R_2N\text{-}(CH_2)_a\text{-}NR_3R_4\,(HF)_b\,(H_2PO_3F)_c \qquad (I)$$

in der $R_1$, $R_2$, $R_3$, $R_4$ ein Wasserstoffatom, einen geradkettigen, verzweigten oder cyclischen, gesättigten oder ungesättigten Alkylrest mit 1 bis 30 C-Atomen bedeuten, wobei diese gegebenenfalls noch durch Alkyl-, Alkoxy- oder Hydroxylgruppen substituiert sein können und a den Wert 1 bis 6, b den Wert 0 oder 1, sowie c den Wert 1 (für b = 0 oder 1) oder 2 (für b = 0) annehmen kann, wobei mindestens einer der Reste $R_1$-$R_4$ nicht Wasserstoff ist, und einer der Reste $R_1$-$R_4$ auch eine Gruppe $(H_2PO_3F)x\ R_1R_2N\text{-}(CH_2)_a$ - oder $(HF)x\ R_1R_2N\text{-}(CH_2)_a$- darstellen kann, worin a, $R_1$ und $R_2$ die obige Bedeutung haben, sowie Verfahren zu deren Herstellung und deren Verwendung in Zahnpflegemitteln.

Die beanspruchten Substanzen zeichnen sich durch eine verbesserte Freisetzung von Fluor bei der Anwendung als Zahnpflegemittel aus.

EP 0 248 972 A2

## Ammoniummonofluorophosphate, Verfahren zu ihrer Herstellung und ihre Verwendung

Gegenstand der vorliegenden Erfindung sind spezielle Alkylammoniummonofluorophosphate, Verfahren zu ihrer Herstellung und ihre Verwendung als Zahnpastenadditive zur Kariesinhibierung.

Es ist bekannt, daß Fluoride, die in geringen Mengen Zahnpasten zugesetzt werden, imstande sind die Zahnsubstanz zu härten und dadurch den Zahnverfall und Karies zu verringern. Da die meisten Fluoride, wie auch Flußsäure selbst, recht toxisch sind, ist es weiterhin bekannt, statt dessen Natriummonofluorophosphat ($Na_2PO_3F$) zu verwenden, aus dem Fluorid durch Hydrolyse freigesetzt wird.

Obwohl dieses Salz und eine Reihe weiterer Monofluorophosphate seit langem in großem Umfang als Zahnpastenadditiv verwendet werden, besteht doch das Bedürfnis nach anderen Additiven, die bei der Anwendung als Zahnpasta in zeitlich unterschiedlichem Maße Fluorid freisetzen.
Es stellte sich daher die Aufgabe neue, als Zahnpastenadditive besser geeignete, Fluorid abgebende Verbindungen zu finden.

Überraschenderweise wurde nun gefunden, daß Ammoniummonofluorophosphate der allgemeinen Formel I

$$R_1R_2N-(CH_2)_a-NR_3R_4\ (HF)_b\ (H_2PO_3F)_c \quad (I)$$

in der $R_1$, $R_2$, $R_3$, $R_4$ ein Wasserstoffatom, einen geradkettigen, verzweigten oder cyclischen, gesättigten oder ungesättigten Alkylrest mit 1 bis 30 C-Atomen bedeuten, wobei diese gegebenenfalls noch durch Alkyl-, Alkoxy- oder Hydroxylgruppen substituiert sein können und a den Wert 1 bis 6, b den Wert 0 oder 1, sowie c den Wert 1 (für b = 0 oder 1) oder 2 (für b = 0) annehmen kann, wobei mindestens einer der Reste $R_1$-$R_4$ nicht Wasserstoff ist, und einer der Reste $R_1$-$R_4$ auch eine Gruppe $(H_2PO_3F)\ R_1R_2N-(CH_2)_a-$ oder $(HF)\ R_1R_2N-(CH_2)_a-$ darstellen kann, worin a, $R_1$ und $R_2$ die obige Bedeutung haben, in diesem Sinne verwendet werden können.
Insbesondere sind unsymmetrische Verbindungen bevorzugt, in denen die Gruppe $NR_1R_2$ eine andere Bedeutung hat als $NR_3NR_4$.

Für die erfindungsgemäße Verwendung als Zahnpastaadditiv ist es wichtig, daß die hydrolytische Spaltung der Phosphor-Fluor-Bindung bei diesen Verbindungen im Mundbereich langsam aber kontinuierlich abläuft, so daß eine niedere, jedoch konstante Konzentration an freien Fluoriden erhalten wird. Durch die zusätzliche Kombination von Ammoniumfluoriden und Ammoniummonofluorophosphaten am selben Kation ist die Möglichkeit gegeben, gleichzeitig sowohl einen rasch wirkenden Schutz durch hydrolytische Spaltung der Ammoniumfluoridgruppe, als auch eine länger andauernde Schutzwirkung durch die deutlich langsamere Hydrolyse der Phosphor-Fluor-Bindung zu erzielen.

Durch Variation der Reste R ergibt sich ein breites Spektrum

an Anwendungsmöglichkeiten, wie beispielsweise die Kombination von Resten mit guter Hafteigenschaft auf der Mundschleimhaut mit Resten variabler Hydrophobie und damit unterschiedlich schneller Fluorid-Freisetzung.

Mit Hilfe der erfindungsgemäßen neuen Verbindungen können deshalb erstmals die verschiedenen Ansprüche an Zahnpasten mit beispielsweise großer Langzeitwirkung (für abends), sowie mit mäßig schneller Fluoridfreisetzung bei gleichzeitig guter Haftung (für morgens und mittags), für den Anwender in einfacher Art und Weise realisiert werden.

Aus der USP 3,507,918 sind bereits gesättigte Amine enthaltende symmetrische Mono- und Di(alkylammonium)monofluorophosphate bekannt, die als Öl-lösliche Schmiermitteladditive verwendet werden. Niedermolekulare Verbindungen dieser Art werden weiterhin als Stickstoff- und Phosphorlieferanten zur Bodenkonservierung und Düngung verwendet. Entsprechende Salze von Diaminen werden in dieser Patentschrift nicht erwähnt.

In der USP 4,105,759 und der DOS 28 12 117 werden neuartige symmetrische Bis(alkylammonium)monofluorophosphate der allgemeinen Formel $(RNH_3)_2PO_3F$, in der R einen gesättigten oder ungesättigten Alkylrest mit 8 bis 18 C-Atomen bedeutet. Unsymmetrische Verbindungen oder Monofluorophosphate von Diaminen werden in dieser Anmeldung nicht erwähnt.

In der USP 4,011,310 werden neue Alkylammoniumfluorophosphate zur Dentalprophylaxe beschrieben. Dabei handelt es sich um Mono- bzw. Difluorophosphate von primären, sekundären oder tertiären Monoaminen und primären Alkylendiaminen.
Überraschenderweise wurde nun gefunden, daß die bisher nicht beschriebenen Alkylendiamine bei denen mindestens eine der Aminogruppen sekundär oder tertiär ist, d.h. einen oder zwei

zusätzliche Alkylgruppen trägt, Dimonofluorophosphate bilden, welche aufgrund ihres polaren Charakters für die Verwendung als Zahnpastenadditiv besonders geeignet sind.

Die Verbindungen der allgemeinen Formel I werden in einfacher Weise durch Umsetzung der Monofluorophosphorsäure oder ihrer Salze, vorzugsweise des Dinatriummonofluorophosphates, des Calciummonofluorophosphates oder des Bariummonofluorophosphates, mit den entsprechenden Aminen bzw. ihren Salzen, vorzugsweise den Hydrochloriden oder Sulfaten, erhalten.

Falls das gegebenenfalls als Nebenprodukt entstehende Salz (z.B. NaCl oder Natriumsulfat) bei der jeweiligen Formulierung der Zahnpasta nicht stört, wird bevorzugt das Natriummonofluorophosphat mit dem entsprechenden Ammoniumchlorid bzw. Ammoniumsulfat umgesetzt, wobei überraschenderweise eine nahezu quantitative Umsalzung unter Bildung der Ammoniummonofluorophosphate stattfindet. Die Reaktion wird durch Zusammenmischen der Komponenten in einem Lösemittel bei Raumtemperatur oder bis zum Siedepunkt des Lösemittels erhöhter Temperatur durchgeführt. Als Lösemittel kommen sowohl Wasser als auch polare organische Lösemittel wie Aceton, Methylisobutylketon, niedere Alkohole wie Methanol, Ethanol, Propanol, Butanol, aber auch Gemische dieser Lösemittel in Frage. Gegebenenfalls kann durch Zusatz von unpolaren organischen Lösemitteln wie Toluol die Löslichkeit der Verbindungen der Formel I und/oder der Nebenprodukte verändert werden und damit die gesuchte Verbindung I in höherer Reinheit und Ausbeute entstehen.

Aufgrund der guten physiologischen Verträglichkeit sowie dem Löslichkeitsverhalten der Edukte und Produkte wird als Lösemittel Wasser oder Ethanol, oder Gemische dieser beiden Lösemittel bevorzugt. Bei Verwendung dieser beiden Lösemittel ist eine weitere Aufarbeitung der Reaktionsprodukte überflüssig,

da diese direkt als Lösung für die entsprechenden Formulierungen zur Anwendung gelangen können. Die erfindungsgemäßen Verbindungen können jedoch auch in fester Form erhalten werden, indem nach erfolgter Umsetzung das Lösemittel abgezogen und aus dem Rückstand das Ammoniummonofluorophosphat mit einem wasserfreien organischen Lösemittel extrahiert wird, wobei das Natriumchlorid bzw. Natriumsulfat zurück bleibt, und durch Abziehen des organischen Lösemittels das reine Ammoniummonofluorophosphat gemäß Formel I gewonnen werden kann. Um ein übermäßiges Schäumen zu vermeiden, empfiehlt es sich beim Abziehen des Lösemittels einen entsprechenden, bei der Verwendung als Zahnpastenadditiv nicht störenden Entschäumer, zuzusetzen.

Zur leichten Gewinnung von in Wasser gut löslichen Ammoniummonofluorophosphaten gemäß Formel I, ist es ebenso möglich die entsprechende Umsalzung unter Bildung eines in Wasser schwer löslichen Salzes vorzunehmen. Vorzugsweise wird dabei Calcium- oder Bariummonofluorophosphat mit dem entsprechenden Ammoniumsulfat unter Bildung von Calcium- oder Bariumsulfat zur Reaktion gebracht. Letztere lassen sich aufgrund ihrer geringen Löslichkeit nach dem Fachmann an sich bekannten Methoden leicht von der wässrigen Lösung des erfindungsgemäßen Ammoniummonofluorophosphates abtrennen und dieses durch Abziehen des Lösemittels nach der oben beschriebenen Verfahrensweise gewinnen.

In Wasser schwer lösliche Verbindungen gemäß Formel I können direkt nach Beendigung der Umsalzung durch Filtration, Zentrifugation oder andere dem Fachmann an sich bekannte Methoden von der wässrigen Lösung abgetrennt werden. Die weiteren Reaktionsprodukte, wie beispielsweise Natriumchlorid oder Natriumsulfat, verbleiben dabei in Lösung.
Eine zusätzliche Reinigung des Produktes kann in vielen Fällen durch Umkristallisation, beispielsweise aus einem

niederen Alkohol, erfolgen.



Weiterhin wurde gefunden, daß man durch Umsetzen von Dinatriummonofluorophosphat mit sauren, vorzugsweise stark sauren Ionenaustauschern in wäßriger Lösung Monofluorophosphorsäure in hoher Reinheit erhalten kann, die bei sofortiger Umsetzung mit den entsprechenden Aminen reine Ammoniummonofluorophosphate ergibt. Da bei der Reaktion eine erhebliche Neutralisationswärme frei wird, muß die Lösung gekühlt werden, wobei Reaktionstemperaturen von 0 bis 60 °C, vorzugsweise 10 bis 30 °C und insbesondere 15 bis 20 °C, bevorzugt werden. Das Amin bzw. Amingemisch wird dabei als Lösung oder Suspension in Wasser oder einem mit Wasser mischbaren organischen Lösemittel, wie beispielsweise Ethanol, Aceton, Methylisobutylketon, einem niederen Alkohol oder einer Mischung derselben, vorgelegt. Zur Vermeidung lokaler Überhitzung muß die Zugabe des Ionenaustauschereluates unter kräftigem Rühren erfolgen. Nach erfolgter Zugabe des stöchiometrische Mengen Monofluorophosphorsäure enthaltenden Ionenaustauschereluates, wird die Mischung zur vollständigen Umsetzung noch eine entsprechende Zeit weitergerührt. Die Reaktionsdauer liegt zwischen 15 Minuten bis 6 Stunden, vorzugsweise zwischen 1 und 3 Stunden. Die erfindungsgemäßen Ammoniummonofluorophosphate werden als Lösung oder, durch Abziehen des Lösemittels im Vakuum und Umkristallisation des Rückstandes in einem geeigneten Lösemittel, wie beispielsweise in einem niederen Alkohol oder Toluol, als Feststoff gewonnen. Die Produkte können anschließend durch Trocknen im Vakuum von anhaftenden letzten Spuren der Lösemittel befreit werden.

Ebenso kann die Bildung der erfindungsgemäßen Verbindungen direkt aus käuflicher Monofluorophosphorsäure und den entsprechenden Aminen erfolgen. Dabei kann eine zusätzliche Reinigung, beispielsweise durch Umkristallisation, erforderlich werden.

Die erfindungsgemäßen Ammoniummonofluorophosphate der allgemeinen Formel I sind feste bis wachsartige Substanzen, die je nach Art der Substituenten eine merkliche bis sehr gute Löslichkeit in Wasser besitzen. Der pH-Wert einer 1%-igen wäßrigen Lösung oder Dispersion dieser Verbindungen liegt bei pH 5-7. In Alkoholen sind die erfindungsgemäßen Monofluorophosphate im allgemeinen in der Wärme gut löslich.

Als Zahnpastenadditive werden die erfindungsgemäßen Verbindungen in einer Menge von 100 bis 5000 ppm, vorzugsweise 500 bis 2000 ppm Fluoridäquivalenten, d.h. bis etwa 10 Gew.% zugesetzt.

Unter Alkylgruppen im Sinne der Erfindung werden Gruppen mit bis zu 30 C-Atomen, insbesondere 10-22 C-Atomen verstanden. Diese Gruppen können gegebenenfalls 1 oder 2 Doppelbindungen enthalten.

Cycloalkylgruppen können Monocyclen mit 5-8 C-Atomen, insbesondere c-Pentyl und c-Hexyl sein, aber auch Bicyclen, wie Tetrahydronaphthalin und Decalin.

Soweit Alkyl- oder Alkoxygruppen als Substituenten der vorstehenden Reste auftreten, sind Gruppen mit 1-6 C-Atomen, insbesondere Methyl und Ethyl bzw. Methoxy und Ethoxy bevorzugt.

Die erfindungsgemäß hergestellten Substanzen können auch als Mischungen aller unter Formel I beschriebenen Verbindungen vorliegen.

## Beispiel 1

36 g Natriummonofluorophosphat werden in 500 ml $H_2O$ gelöst und über eine Säule mit Kationenaustauscher (400 ml Lewatit S 100) gegeben. Die entstehende wäßrige Monofluorophosphorsäure wird bei 40° C unter kräftigem Rühren in 114,7 g Bis-(hydroxyethyl)aminopropylen-N-hydroxyethyl-octadecylamin eingetragen.
Die Umsetzung zu Bis-(hydroxyethyl)aminopropylen-N-hydroxyethyl-octadecylammoniummonofluorophosphat erfolgt quantitativ.

Die wäßrige Lösung wird bei 80 - 120 mbar und 40° C aufkonzentriert und das Bis-(hydroxyethyl)aminopropylen-N-hydroxyethyloctadecylammoniummonofluorophosphat mit einem organischen Lösemittel wie beispielsweise Aceton ausgefällt.

Die anschließende Trocknung im Vakuum bei 40° C ergibt 123,6 g Bis-(hydroxyethyl)aminopropylen-N-hydroxyethyl-octadecylammoniummonofluorophosphat, das sind 88,5 % der Theorie bezogen auf eingesetztes Diamin.

## Beispiel 2

45,9 g Bis-(hydroxyethyl)aminopropylen-N-hydroxyethyl-octadecylamin werden innerhalb von 15 Minuten unter starkem Rühren und Kühlen auf 30° C mit 40 ml 5N $H_2SO_4$ versetzt. Unter fortdauerndem Rühren werden 13,8 g Calciummonofluorophosphat zudosiert und 1 Stunde bei 40° C nachgerührt.

Danach wird das ausgefallene Calciumsulfat mittels Filtration abgetrennt und mit wenig $H_2O$ nachgewaschen. Das gesamte wäßrige Filtrat wird im Vakuum bei 40° C bis zur Trockene eingeengt, dann der wachsartige Rückstand in Methanol aufgelöst, nochmals filtriert und das Bis-(hydroxyethyl)aminopropylen-N-hydroxyethyl-octadecylammoniummonofluorophosphat in einem organischen Lösemittel wie beispielsweise Aceton oder Ethylacetat unter starkem Rühren ausgefällt.

Die anschließende Trocknung im Vakuum bei 40° C ergibt 50 g Bis-(hydroxyethyl)aminopropylen-N-hydroxyethyl-octadecylammoniummonofluorophosphat, das sind 89,5 % der Theorie bezogen auf eingesetztes Diamin.

## Beispiel 3

64,9 g N-Oleyl-1,3-diaminopropan werden mit 500 ml $H_2O$ versetzt und innerhalb von 15 Minuten unter starkem Rühren mit 200 ml 2n HCl neutralisiert. Es wird mit 1000 ml $H_2O$ verdünnt, auf 40 $^\circ$C erwärmt und dann unter fortdauerndem Rühren 28,8 g $Na_2PO_3F$, gelöst in 100 ml $H_2O$, zugesetzt.
Die Lösung wird weitere 2 Stunden bei 40 $^\circ$C gerührt, danach mit 200 ml Aceton versetzt und das schaumartige Produkt mittels Filtration abgetrennt und kurz mit $H_2O$ gewaschen.

Die anschließende Trocknung im Vakuum bei 40 $^\circ$C ergab 73,8 g N-Oleyl-1,3-diammoniumpropanmonofluorophosphat, d.s. 86,9 % der Theorie, bezogen auf eingesetzte Edukte.

Mit Wasser ergibt N-Oleyl-1,3-diammoniumpropanmonofluorophosphat bei 20 $^\circ$C eine stabile Emulsion, die Löslichkeit in Glycerin liegt über 10 %.

## Beispiel 4

45,8 g N,N',N'-Tris(hydroxyethyl)-N-Talgfett-1,3-diaminopropan werden mit 1000 ml Ethanol versetzt und innerhalb von 15 Minuten unter starkem Rühren und Kühlen auf 30 $^\circ$C mit einer Lösung von 10 g Monofluorophosphorsäure in 500 ml Ethanol versetzt. Das farblose Reaktionsprodukt wird abgesaugt und mit wenig Ethanol gewaschen.

Die anschließende Trocknung im Vakuum bei 40 $^\circ$C ergab 43,7 g N,N',N'-Tris(hydroxyethyl)-N-Talgfett-1,3-diammoniumpropanmonofluorophosphat, d.s. 78,3 % der Theorie, bezogen auf eingesetzte Edukte.

## Beispiel 5

29,8 g N-Lauryl-1,3-diaminopropan werden mit 1000 ml Ethanol versetzt und innerhalb von 15 Minuten unter starkem Rühren und Kühlen auf 30 °C mit einer Lösung von 10 g Monofluorophosphorsäure in 500 ml Ethanol versetzt. Das Reaktionsprodukt wird abgesaugt und mit wenig Ethanol gewaschen.

Die anschließende Trocknung im Vakuum bei 40 °C ergab 45,4 g N-Lauryl-1,3-diammoniumpropan-bis(monofluorophosphat), d.s. 91,2 % der Theorie, bezogen auf eingesetzte Edukte.

## Beispiel 6

33,7 g Pentakis(hydroxyethyl)-diethylentriamin werden mit 1000 ml Ethanol versetzt und innerhalb von 15 Minuten unter starkem Rühren und Kühlen auf 30 °C mit einer Lösung von 30 g Monofluorophosphorsäure in 1000 ml Ethanol versetzt. Das Reaktionsprodukt wird abgesaugt und mit wenig Ethanol gewaschen.

Die anschließende Trocknung im Vakuum bei 40 °C ergab 57,4 g Pentakis(hydroxyethyl)-diethylentriammonium-tris(monofluorophosphat), d.s. 90,1 % der Theorie, bezogen auf eingesetzte Edukte.

## Beispiel 7

34,4 g N-Oleyl-1,3-diaminopropan-monohydrogenfluorid werden mit 1000 ml Ethanol versetzt und innerhalb von 15 Minuten unter starkem Rühren und Kühlen auf 30 °C mit einer Lösung von 10 g Monofluorophosphorsäure in 500 ml Ethanol versetzt. Das Reaktionsprodukt wird abgesaugt und mit wenig Ethanol gewaschen.

Die anschließende Trocknung im Vakuum bei 40 °C ergab 41,6 g N-Oleyl-1,3-diammoniumpropan-monofluorido-monofluorophosphat, d.s. 93,8 % der Theorie, bezogen auf eingesetzte Edukte.

In analoger Weise wurden entsprechend den Beispielen 1-7 die folgenden Verbindungen bzw. ihre Monohydrogenfluoride zu den entsprechenden Monofluorophosphaten bzw. gemischten Fluoridomonofluorophosphaten umgesetzt:

- N,N'-Pentamethyl-N-talgfett-1,3-propandiammoniumchlorid
- N-Talgfett-1,3-diaminopropan
- N-Talgfett-1,3-diaminopropandioleat
- Talgfettpropylendiamin x 10 Ethoxygruppen

# Patentansprüche

1. Ammoniummonofluorophosphate der allgemeinen Formel I

$$R_1R_2N-(CH_2)_a-NR_3R_4\ (HF)_b\ (H_2PO_3F)_c\ (I)$$

in der $R_1$, $R_2$, $R_3$, $R_4$ ein Wasserstoffatom, einen geradkettigen, verzweigten oder cyclischen, gesättigten oder ungesättigten Alkylrest mit 1 bis 30 C-Atomen bedeuten, wobei diese gegebenenfalls noch durch Alkyl-, Alkoxy- oder Hydroxylgruppen substituiert sein können und a den Wert 1 bis 6, b den Wert 0 oder 1, sowie c den Wert 1 (für b = 0 oder 1) oder 2 (für b = 0) annehmen kann, wobei mindestens einer der Reste $R_1$-$R_4$ nicht Wasserstoff ist, und einer der Reste $R_1$-$R_4$ auch eine Gruppe $(H_2PO_3F)x\ R_1R_2N-(CH_2)_a-$ oder $(HF)x\ R_1R_2N-(CH_2)_a-$ darstellen kann, worin a, $R_1$ und $R_2$ die obige Bedeutung haben.

2. Verfahren zur Herstellung von Ammoniummonofluorophosphaten gemäß Formel I, dadurch gekennzeichnet, daß man

a) ein Salz der Monofluorophosphorsäure in einem Lösemittel mit einem oder mehreren Ammoniumsalzen der allgemeinen Formel II,

$$R_1R_2N-(CH_2)_a-NR_3R_4 \quad x\ HX \qquad (II)$$

in der $R_1$, $R_2$, $R_3$, $R_4$ die oben genannten Bedeutungen haben und X das Anion einer Mineralsäure darstellt, umsetzt oder

b) ein Salz der Monofluorophosphorsäure mit einem sauren Ionenaustauscher in wäßriger Lösung in die freie Monofluorophosphorsäure überführt und diese sofort mit einer Lösung des oder der entsprechenden Amine der allgemeinen Formel II' neutralisiert, oder

$$R_1R_2N-(CH_2)_a-NR_3R_4 \qquad (II')$$

c) freie Monofluorophosphorsäure mit einer Lösung des oder der entsprechenden Amine der allgemeinen Formel II' neutralisiert

und die entstehende Verbindung der allgemeinen Formel I isoliert.

3. Zahnpasta enthaltend ein oder mehrere Ammoniummonofluorophosphate gemäß Anspruch 1 als Zahnpastenadditiv.

4. Zahnpasta enthaltend Verbindungen gemäß Anspruch I in Mischung mit für diesen Zweck geeigneten Ammoniummonofluorphosphaten und/oder Ammoniumfluoriden und/oder Alkylammoniumfluoriden.